(19) Europäisches Patentamt
European Patent Office
Office européen des brevets

(11) **EP 3 149 481 B1**

(12) **EUROPEAN PATENT SPECIFICATION**

(45) Date of publication and mention
of the grant of the patent:
**09.01.2019 Bulletin 2019/02**

(21) Application number: **15727610.6**

(22) Date of filing: **29.05.2015**

(51) Int Cl.:
**G01N 33/574** (2006.01)

(86) International application number:
**PCT/EP2015/061922**

(87) International publication number:
**WO 2015/181343 (03.12.2015 Gazette 2015/48)**

(54) **MULTIPLEX ASSAY FOR IMPROVED SCORING OF TUMOR TISSUES STAINED FOR PD-L1**

MULTIPLEXASSAY ZUR VERBESSERTEN EINTEILUNG VON FÜR PD-L1 EINGEFÄRBTEN TUMORGEWEBEN

ANALYSE MULTIPLEXE POUR UNE MEILLEURE DÉTERMINATION DES SCORES DE TISSUS TUMORAUX COLORÉS POUR PD-L1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2014 US 201462005701 P**

(43) Date of publication of application:
**05.04.2017 Bulletin 2017/14**

(73) Proprietor: **Ventana Medical Systems, Inc.**
**Tucson, Arizona 85755 (US)**

(72) Inventors:
• **NITTA, Hiro**
**Tucson, Arizona 85718 (US)**
• **VENNAPUSA, Bharathi**
**Tucson, Arizona 85737 (US)**
• **DENNIS, Eslie**
**Tucson, Arizona 85704 (US)**

(74) Representative: **Burger, Alexander et al**
**Roche Diagnostics GmbH**
**Patent Department (LPP.....6164)**
**P.O.Box 11 52**
**82372 Penzberg (DE)**

(56) References cited:
• **Elmer Perkin: "MULTIPLEX TISSUE BIOMARKERS IN CONTEXT", , 6 May 2014 (2014-05-06), XP55433617, Retrieved from the Internet: URL:https://www.perkinelmer.com/lab-soluti ons/resources/docs/FLY_Opal-Multiplex-Stai ning.pdf [retrieved on 2017-12-08]**
• **S. LYFORD-PIKE ET AL: "Evidence for a Role of the PD-1:PD-L1 Pathway in Immune Resistance of HPV-Associated Head and Neck Squamous Cell Carcinoma", CANCER RESEARCH, vol. 73, no. 6, 3 January 2013 (2013-01-03), pages 1733-1741, XP55179352, ISSN: 0008-5472, DOI: 10.1158/0008-5472.CAN-12-2384**
• **R C Lloyd ET AL: "Phenotyping immune cells in-situ. An investigation of the spatial heterogeneity of specific immune cell phenotypes in the tumour microenvironment", , 8 March 2014 (2014-03-08), XP55433943, Retrieved from the Internet: URL:www.poster-submission.com/cdrom/downl o ad_poster/37/27814/1062P [retrieved on 2017-12-11]**
• **GHEBEH H ET AL: "The B7-H1 (PD-L1) T lymphocyte-stimulatory molecule is expressed in breast cancer patients with infiltrating ductal carcinoma: correlation with important high-risk prognostic factors", NEOPLASIA, NEOPLASIA PRESS, ANN ARBOR, MI, US, vol. 8, no. 3, 1 March 2006 (2006-03-01), pages 190-198, XP008091677, ISSN: 1522-8002, DOI: 10.1593/NEO.05733**

- GHEBEH HAZEM ET AL: "FOXP3+ Tregs and B7-H1+/PD-1+ T lymphocytes co-infiltrate the tumor tissues of high-risk breast cancer patients: Implication for immunotherapy", BMC CANCER, BIOMED CENTRAL, LONDON, GB, vol. 8, no. 1, 23 February 2008 (2008-02-23), page 57, XP021034690, ISSN: 1471-2407
- O. K. AFANASIEV ET AL: "Merkel Polyomavirus-Specific T Cells Fluctuate with Merkel Cell Carcinoma Burden and Express Therapeutically Targetable PD-1 and Tim-3 Exhaustion Markers", CLINICAL CANCER RESEARCH, vol. 19, no. 19, 1 October 2013 (2013-10-01), pages 5351-5360, XP055207749, ISSN: 1078-0432, DOI: 10.1158/1078-0432.CCR-13-0035
- J Powderly ET AL: "Biomarkers and Associations With the Clinical Activity of PD-L1 Blockade in a MPDL3280A Study", , 1 January 2013 (2013-01-01), XP055207634, Retrieved from the Internet: URL:http://carolinabiooncology.org/wp-content/uploads/ASCO-2013-Presentation-Anti-PD L-1-Biomarkers.pdf [retrieved on 2015-08-13]
- YOSHIKO IWAI ET AL: "PD-1 blockade inhibits hematogenous spread of poorly immunogenic tumor cells by enhanced recruitment of effector T cells.", INTERNATIONAL IMMUNOLOGY, vol. 17, no. 2, 1 February 2005 (2005-02-01), pages 133-44, XP055142463, ISSN: 0953-8178, DOI: dxh194
- Elmer Perkin: "MULTIPLEX TISSUE BIOMARKERS IN CONTEXT", , 6 May 2014 (2014-05-06), XP55433617, Retrieved from the Internet: URL:https://www.perkinelmer.com/lab-soluti ons/resources/docs/FLY_Opal-Multiplex-Stai ning.pdf [retrieved on 2017-12-08]
- S. LYFORD-PIKE ET AL: "Evidence for a Role of the PD-1:PD-L1 Pathway in Immune Resistance of HPV-Associated Head and Neck Squamous Cell Carcinoma", CANCER RESEARCH, vol. 73, no. 6, 3 January 2013 (2013-01-03), pages 1733-1741, XP55179352, ISSN: 0008-5472, DOI: 10.1158/0008-5472.CAN-12-2384
- R C Lloyd ET AL: "Phenotyping immune cells in-situ. An investigation of the spatial heterogeneity of specific immune cell phenotypes in the tumour microenvironment", , 8 March 2014 (2014-03-08), XP55433943, Retrieved from the Internet: URL:www.poster-submission.com/cdrom/downl o ad_poster/37/27814/1062P [retrieved on 2017-12-11]
- Elmer Perkin: "MULTIPLEX TISSUE BIOMARKERS IN CONTEXT", , 6 May 2014 (2014-05-06), XP055433617, Retrieved from the Internet: URL:https://www.perkinelmer.com/lab-soluti ons/resources/docs/FLY_Opal-Multiplex-Stai ning.pdf [retrieved on 2017-12-08]
- S. LYFORD-PIKE ET AL: "Evidence for a Role of the PD-1:PD-L1 Pathway in Immune Resistance of HPV-Associated Head and Neck Squamous Cell Carcinoma", CANCER RESEARCH, vol. 73, no. 6, 3 January 2013 (2013-01-03), pages 1733-1741, XP055179352, ISSN: 0008-5472, DOI: 10.1158/0008-5472.CAN-12-2384
- R C Lloyd ET AL: "Phenotyping immune cells in-situ. An investigation of the spatial heterogeneity of specific immune cell phenotypes in the tumour microenvironment", , 8 March 2014 (2014-03-08), XP055433943, Retrieved from the Internet: URL:www.poster-submission.com/cdrom/downl o ad_poster/37/27814/1062P [retrieved on 2017-12-11]

**Description**

**BACKGROUND OF THE INVENTION**

*Field of the Invention*

The present disclosure relates to methods for histochemically scoring PD-L1 expression in tumor tissues.

*Description of related art*

Programmed death 1 (PD-1) is a member of the CD28 family of receptors, which includes CD28, CTLA-4, ICOS, PD-1, and BTLA. Two cell surface glycoprotein ligands for PD-1 have been identified, PD-L1 and PD-L2, and have been shown to downregulate T cell activation and cytokine secretion upon binding to PD-1 (Freeman et al., J Exp Med 192:1027-34 (2000); Latchman et al., Nat Immunol 2:261-8 (2001); Carter et al., Eur J Immunol 32:634-43 (2002); Ohigashi et al., Clin Cancer Res 11:2947-53 (2005)). Both PD-L1 (B7-H1) and PD-L2 (B7-DC) are B7 homologs that bind to PD-1, but do not bind to other CD28 family members.

The PD-L1-PD1 pathway is involved in the negative regulation of some immune responses and may play an important role in the regulation of peripheral tolerance. Interaction of PD-L1 with PD1 results in inhibition of TCR-mediated proliferation and cytokine production. PD-L1 has been suggested to play a role in tumor immunity by increasing apoptosis of antigen-specific T-cell clones (Dong et al. Nat Med 8:793-800 (2002)). Indeed, PD-L1 expression has been found in several murine and human cancers, including human lung, ovarian and colon carcinoma and various myelomas (Iwai et al. PNAS 99:12293-7 (2002); Ohigashi et al. Clin Cancer Res 11:2947-53 (2005)). Thus, measuring the amount of PD-L1 protein in biological samples may aid in the early detection of cancer pathologies and may help assess the efficacy and durability of investigational drugs that inhibit the binding of the PD-L1 protein.

Ghebeh et al. (Neoplasia. 2006 Mar;8(3):190-8) disclose chromogenic immunohistochemistry (IHC) assays wherein snap-frozen, acetone-fixed breast cancer tissue samples are double-stained in separate assays for B7-H1 (PD-L1) and one of FoxP3, CD3, CD 4, CD 8 and pan-Cytokeratin.

Ghebeh et al. (BMC Cancer. 2008 Feb 23;8:57) are also concerned with chromogenic IHC double-staining assays using, amongst others, anti-B7-H1 (PD-L1) and anti-FoxP3 antibodies, and anti-B7-H1 and anti-CD8 antibodies.

Afanasiev et al. (Clin Cancer Res. 2013 Oct 1;19(19):5351-60) disclose the analysis of PD-L1 protein expression in Merkel cell carcinoma (MCC) samples. Formalin-fixed paraffin-embedded MCC samples are analyzed in a chromogenic single-marker IHC assay using an anti-PD-Ll antibody.

Powderly *et al.* (http://carolinabiooncology.org/wp-content/uploads/ASCO-2013-Presentation-Anti-PD-L-1Biomarkers.pdf) also present PD-L1 expression data that were obtained in chromogenic single-marker IHC assays.

Iwai et al. (Int Immunol. 2005 Feb;17(2):133-44) disclose data from fluorescence IHC assays with cryosections of liver tissue that are double-stained for PD-L1 and CD11b. Also disclosed are fluorescence assays that show PD-L1 staining and, in addition, counterstained nuclei.

PerkinElmer (https://www.perkinelmer.comllab-solutions/resources/docs/ FLY_Opal-Multiplex-Staining.pdf) is concerned with TSA-based fluorescence multiplex assays and presents one photomicrograph of a breast cancer tissue microarray with different fluorescence signals for HER2, ER, PR, Ki-67 and nuclei, and another photomicrograph of a breast cancer tissue section showing different fluorescence signals for CD8, CD4, CD20, cytokeratin and nuclei.

Lyford-Pike et al. (Cancer Res. 2013 Mar 15;73(6)) disclose the analysis of PD-L1 and CD4 protein expression in formalin-fixed paraffin-embedded tissue samples from human papillomavirus-associated head and neck squamous cell carcinomas (HPV-HNSCC). In particular, the samples are analyzed in separate chromogenic single-marker IHC assays using an anti-PD-Ll antibody or antibodies against various immune cell-specific markers such as CD3, CD4, CD68 or CD1a.

Lloyd *et al.* (www.poster-submission.com/cdrom/download_poster/ 37/27814/1062P) discuss multispectral imaging and linear unmixing of signals in a multiplex chromogenic IHC assay with a tissue microarray stained for CD3, FoxP3 and CD69. No staining of PD-L1 is shown. Instead, the marker is mentioned as an example for fluorescence IHC.

However, the use of PD-L1 protein expression as an accurate predictor for cancer and/or the efficacy of anti-PD-1 and anti-PD-Ll directed therapies remains challenging. For example, many tumor samples show PD-L1 staining in both tumor cells and immune cells. Differentiation of these two cell types may be difficult for pathologists, especially when both are present in the same sample.

**SUMMARY OF INVENTION**

The present invention features multiplex assays for improved scoring of tumor tissues stained with PD-L1. The assays feature PD-L1 staining in a first color plus staining of a differentiating marker specific for tumor cells or immune cells, in a second color, and optionally, a second differentiating marker specific for tumor cells or immune cells, in a third color. The assays of the present invention help to differentiate between the PD-L1 positive tumor cells and the PD-L1 positive immune cells. This may improve the ability of samples to be scored more quickly, accurately, and with a greater degree of reproducibility as compared to scoring samples stained with PD-L1 alone.

In one aspect of the present disclosure is a method of scoring PD-L1 expression in a tumor sample, the method comprising labeling the tumor tissue sample, the labeling comprising contacting the tissue sample with an anti-PD-Ll primary antibody, and contacting the same tissue sample with a primary antibody directed to a tumor cell-specific marker and an antibody directed to an immune cell-specific marker, and visualizing each of the antibodies in the tissue sample with a reagent that generates a detectable signal corresponding to each of the primary antibodies, wherein the anti-PD-Ll antibody has a first detectable signal, the antibody directed to the tumor cell-specific marker has a second detectable signal distinguishable from the first detectable signal, and the antibody directed to an immune cell-specific marker has a third detectable signal distinguishable from the first detectable signal and the second detectable signal, wherein the first, second, and third detectable signals are generated by chromogens; and the method of scoring PD-L1 expression in a tumor sample further comprising scoring PD-L1 expression in tumor cells, immune cells, or both, wherein co-localization of the first and second detectable signals indicates the presence of PD-L1-positive tumor cells and co-localization of the first and third detectable signals indicates the presence of PD-L1-positive immune cells. In some embodiments, the tumor cell-specific marker is selected from the group consisting of a cytokeratin, chromogranin, synaptophysin, CD56, thyroid transcription factor-1 (TTF-1), p53, leukocyte common antigen (LCA), vimentin, and smooth muscle actin, and the immune cell-specific marker is selected from the group consisting of CD3, CD4, CD8, CD19, CD20, CD11c, CD123, CD56, CD14, CD33, or CD66b. In some embodiments, the immune cell-specific marker is a T-cell marker or a B-cell marker. In some embodiments, the antibody directed to the tumor cell-specific marker is a pan-keratin antibody and the antibody directed to the immune cell-specific marker is an anti-CD4 antibody. In some embodiments, the first detectable signal is generated by contacting the tissue sample with a horseradish peroxidase (HRP)-conjugated secondary antibody that recognizes the anti-PD-Ll primary antibody, reacting the HRP with 3,3'-Diaminobenzidine (DAB) to produce a brown color; the second detectable signal is generated by contacting the sample with an alkaline phosphatase (AP) labeled antibody that recognizes the primary antibody directed to a tumor cell-specific marker, reacting the AP with a Fast Red chromogen and naphthol to produce a red color; and the third detectable signal is generated by contacting the sample with a HRP-conjugated secondary antibody that recognizes the primary antibody directed to an immune cell-specific marker, and reacting the HRP with HRP-green chromogen to produce a green color. In some embodiments, the first, second, and/or third detectable signal is an amplified signal, optionally wherein the amplified signal is generated by tyramide signal amplification. In some embodiments, the sample is contacted simultaneously with the primary antibodies or the sample is contacted sequentially with the primary antibodies. In some embodiments, the method further comprises counterstaining the tissue sample, the counterstain producing a fourth detectable signal that is distinguishable from the first, second, and the third detectable signals, optionally the counterstain comprises hematoxylin. In some embodiments, a fifth detectable signal is produced by overlap of the first detectable signal and the second detectable signal. In some embodiments, a sixth detectable signal is produced by overlap of the first detectable signal and the third detectable signal. In some embodiments, the total number of PD-L1 positive and PD-L1-negative tumor cells is quantitated, and the tumor is scored as PD-L1 positive if staining for PD-L1 is detected in greater than about 10% of tumor cells, or the tumor is scored as PD-L1 positive if staining for PD-L1 is detected in greater than about 50% of tumor cells. In some embodiments, the total number of PD-L1 positive and PD-L1-negative immune cells is quantitated, and the tumor is scored as PD-L1 positive if staining for PD-L1 is detected in greater than about 10% of immune cells, or the tumor is scored as PD-L1 positive if staining for PD-L1 is detected in greater than about 50% of immune cells. In some embodiments, PD-L1-positive immune cells, PD-L1 positive tumor cells, and PD-L1 negative tumor cells are quantitated to generate a PD-L1 Value, wherein *PD-L1 Value = PD-L1 positive tumor cells / (PD-L1 negative tumor cells + PD-L1 positive immune cells),* wherein *PD-L1 positive tumor cells* is calculated either by counting the number of cells staining for both the first and second detectable signals or by calculating the area of the tissue sample in which the first detectable signal is associated with the second detectable signal, *PD-L1 negative tumor cells* is calculated either by counting the number of cells staining for the second detectable signal only or by calculating the area of the tissue sample in which the second detectable signal is not associated with the first detectable signal, and *PD-L1 positive immune cells* is calculated either by counting the number of cells staining for both the first and third detectable signals or by calculating the area of the tissue sample in which the first detectable signal is associated with the third detectable signal. In some embodiments, the method further comprises scoring intensity of PD-L1 staining in PD-L1 positive tumor cells and calculating an H score, wherein *H score = 1 * (percentage of PD-L1 positive tumor cells staining at 1+ intensity) + 2 * (percentage of PD-L1 positive tumor cells staining at 2+ intensity) + 3 * (percentage ofPD-L1 positive tumor cells staining at 3+ intensity).*

Any feature or combination of features described herein are included within the scope of the present invention provided that the features included in any such combination are not mutually inconsistent as will be apparent from the context, this specification, and the knowledge of one of ordinary skill in the art. Additional advantages and aspects of the present invention are apparent in the following detailed description and claims.

## BRIEF DESCRIPTION OF THE DRAWINGS

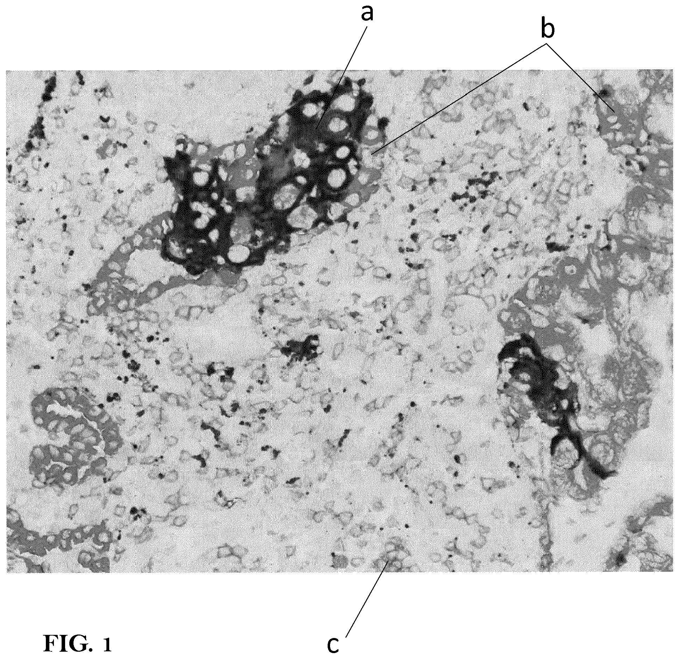

FIG. 1 shows staining of a NSCLC tumor sample. PD-L1 staining is indicated by (a), and would show as brown in a color image. Cytokeratins from pan keratin antibody are indicated by (b), and would be red in a color image. CD4 of immune cells are indicated by (c) and would be green/blue in a color image. Counterstain is diluted hematoxylin.

## DESCRIPTION OF PREFERRED EMBODIMENTS

The present disclosure relates generally to histochemical or cytochemical methods of labeling tumor samples to facilitate scoring of PD-L1 expression in tumor cells, immune cells, or both. Briefly, cells are labeled with binding entities specific for PD-L1 and: (1) at least one tumor cell marker; (2) at least one immune cell marker; or (3) at least one tumor cell marker and at least one immune cell marker. The binding entities are then visualized in the tumor samples by generating at least three distinct detectable signals: a first detectable signal that correlates with the location of PD-L1 binding entity; a second detectable signal that correlates with the location of the tumor cell-specific binding entity; and a third detectable signal that correlates with the location of the immune cell marker. Each of the detectable signals are distinguishable from one another. Optionally, a counterstain may be provided in a fourth detectable signal and/or a fifth detectable signal may be generated from co-localization of any two of the first, second, and third detectable signals.

### Tumor Samples

The present methods are compatible with tumor samples suitable for histochemical or cytochemical analysis, including, for example, fresh frozen, formalin-fixed paraffin-embedded (FFPE)) samples, cytological smears (such as cervical smears), isolates of circulating tumor cells, etc. In a specific aspect, the sample is a FFPE sample of tumor tissue.

### Tumor Cell Markers and Immune Cell Markers

Any marker capable of distinguishing tumor cells from non-tumor cells may be used. Examples of tumor cell-specific biomarkers may include but are not limited to: cytokeratins detectable with the pan keratin antibody (e.g., basic cytokeratins, many of the acidic cytokeratins), other cytokeratins such as cytokeratin 7 (CK7) and cytokeratin 20 (CK20), chromogranin, synaptophysin, CD56, thyroid transcription factor-1 (TTF-1), p53, leukocyte common antigen (LCA), vimentin, smooth muscle actin, or the like (e.g., see Capelozzi, V., J Bras Pneumol. 2009;35(4):375-382).

Any marker capable of distinguishing immune cells from non-immune cells may be used. Examples of immune cell-specific biomarkers may include but are not limited to: CD3, CD4, CD8, CD19, CD20, CD11c, CD123, CD56, CD14, CD33, or CD66b. In one example, a lymphocyte-specific marker is used. For example, a T-cell specific marker, such as CD3, CD4, or CD8, or a B-cell specific marker, such as CD19 or CD20 may be used.

In a specific embodiment, the immune cell marker is CD4 and the tumor cell marker is a cytokeratin detectable by a pan cytokeratin antibody.

### Binding entities

Histochemistry and cytochemistry are techniques often used to identify biomarkers within the context of intact cells by labeling the samples with molecules that bind specifically to the biomarker in a manner that can be visualized on a microscope. Immunohistochemistry (IHC) and immunocytochemistry (ICC) are types of histochemistry and cytochemistry that use antibodies to label the biomarkers. *In situ* hybridization (ISH) is a type of histochemistry or cytochemistry that uses nucleic acid probes to label specific nucleotide sequences in the tissue or cell sample. By identifying the biomarker in the context of a tissue environment or cellular environment, spatial relationships between the biomarkers and other morphological or molecular features of the cell or tissue sample can be elucidated, which may reveal information that is not apparent from other molecular or cellular techniques.

As used herein, the term "binding entity" shall refer to any compound or composition that is capable of specifically binding to a specific molecular structure in a tumor sample suitable for histochemical or cytochemical analysis. Examples include antibodies and antigen binding fragments thereof, as well as engineered specific binding structures, including ADNECTINs (scaffold based on 10th FN3 fibronectin; Bristol-Myers-Squibb Co.), AFFIBODYs (scaffold based on Z domain of protein A from *S. aureus*; Affibody AB, Solna, Sweden), AVIMERs (scaffold based on domain A/LDL receptor; Amgen, Thousand Oaks, CA), dAbs (scaffold based on VH or VL antibody domain; GlaxoSmithKline PLC, Cambridge, UK), DARPins (scaffold based on Ankyrin repeat proteins; Molecular Partners AG, Zürich, CH), ANTICALINs (scaffold based on lipocalins; Pieris AG, Freising, DE), NANOBODYs (scaffold based on VHH (camelid Ig); Ablynx N/V, Ghent, BE), TRANS-BODYs (scaffold based on Transferrin; Pfizer Inc., New York, NY), SMIPs (Emergent Biosolutions, Inc., Rockville, MD), and TETRANECTINs (scaffold based on C-type lectin domain (CTLD), tetranectin; Borean Pharma A/S, Aarhus, DK). Descriptions of such engineered specific binding structures are reviewed by Wurch et al., Development of Novel Protein Scaffolds as Alternatives to Whole Antibodies for Imaging and Therapy: Status on Discovery Research and Clinical Validation, Current Pharmaceutical Biotechnology, Vol. 9, pp. 502-509 (2008).

In an example, the binding entities are antibodies or antigen-binding fragments thereof. As used herein, the term "antibody" refers to any form of antibody that exhibits the desired biological or binding activity. Thus, it is used in the broadest sense and specifically covers, but is not limited to, monoclonal antibodies (including full length monoclonal antibodies), polyclonal antibodies, multispecific antibodies (e.g., bispecific antibodies), humanized antibodies, fully human antibodies, chimeric antibodies and camelized single domain antibodies.

As used herein, unless otherwise indicated, "antibody fragment" or "antigen binding fragment" refers to antigen binding fragments of antibodies, i.e. antibody fragments that retain the ability to bind specifically to the antigen bound by the full-length antibody, e.g. fragments that retain one or more CDR regions. Examples of antibody binding fragments include, but are not limited to, Fab, Fab', F(ab')2, and Fv fragments; diabodies; linear antibodies; single-chain antibody molecules, e.g., sc-Fv; nanobodies and multispecific antibodies formed from antibody fragments.

Exemplary anti-PD-Ll antibodies include SP263 (fully described in U.S. Provisional Patent Application Serial Number 62/004572, Docket Number 32151 US, and filed May 29, 2014), SP142 (Cat. # M4420, Spring Biosciences, Inc., Pleasanton, CA), and PD-L1 (E1L3N®) XP® Rabbit mAb (Cat. # #13684; Cell Signaling Technologies, Inc., Danvers, MA). For example, the PD-L1 binding entity is SP263 or SP142, the tumor cell-specific binding entity is a pan keratin antibody, and the immune cell specific binding entity is an anti-CD4 antibody. In another example, the PD-L1 binding entity is SP142, the tumor cell-specific binding entity is a pan keratin antibody, and the immune cell specific binding entity is an anti-CD4 antibody.

**Visualization of Specific Binding Entities**

As previously described, the assays of the present invention feature staining of PD-L1 as well as staining of one or more of tumor and immune cell markers to generate a detectable signal that correlates with the location at which an exogenous binding entity has bound to the sample. Histochemical and cytochemical methods of generating detectable signals from exogenous binding entities in samples are well known to one of ordinary skill in the art and typically involve application of one or more labels. Exemplary labels include chromogenic labels, fluorescent labels, luminescent labels, radiometric labels, etc., are used for recognition of the markers or targets (e.g., PD-L1, tumor cell-specific marker, immune cell-specific marker, etc.). Labels are well known to one of ordinary skill in the art and are not limited to the labels described herein. In a specific embodiment, the detectable signals are generated through use of chromogens.

In an embodiment, the label is applied through the use of a secondary antibody. For example, the binding entity may be a primary antibody specific for the PD-L1, immune cell marker, or tumor cell marker. If antibodies derived from different species of animal are used as the primary antibody, a secondary antibody specific for that species of antibody can be used to apply the label. In another example, the primary antibody can be modified to contain a separate moiety that can be bound by a specific binding entity. One example of such a primary antibody is a haptenized antibody (i.e., and antibody modified to contain a specific hapten). Many different haptens are known, so each of the primary antibodies can be modified to contain a different hapten, and different anti-hapten antibodies may be used to specifically label the different primary antibodies.

In another embodiment, the detectable signal may be amplified. As used herein, a signal is "amplified" when more label is deposited per primary antibody than by using a standard primary-secondary antibody arrangement. One commonly used method of amplification is tyramide signal amplification, which is described in Bobrow, M. N., Harris, T. D., Shaughnessy, K. J., and Litt, G. J. (1989) J. Immunol. Methods 125, 279-285. In an exemplary embodiment, a modified form of tyramide signal amplification as described in WO 2013148498 is used.

Referring now to FIG. 1, the present invention features multiplex assays for improved scoring of tumor tissues stained with PD-L1. The assays feature steps for staining PD-L1 in a first color (PD-L1 is shown in brown in FIG. 1), as well as steps for staining a tumor cell-specific marker and/or immune cell-specific marker with a second and/or third differentiating color. For example, FIG. 1 shows PD-L1 stained brown (as indicated by (a)), cytokeratins targeted by pan keratin antibodies (cytokeratins specific for the epithelial cancer cells) stained in red/pink (as indicated by (b)), and CD4 (of immune cells) stained in green/blue (as indicated by (c)). The differentiating colors (red/pink and green/blue) allows one to determine if the PD-L1 that is detected is present in tumor cells or immune cells. Thus, the assays of the present invention help to differentiate between the PD-L1 positive tumor cells and the PD-L1 positive immune cells. This may improve the ability of samples to be scored (manual/ visual, machine/image analysis) more quickly, accurately, and with a greater degree of reproducibility as compared to scoring samples stained with PD-L1 alone.

Table 1 illustrates the use of differentiating markers/colors for differentiating between the PD-L1 positive tumor cells and the PD-L1 positive immune cells. PD-L1 can be detected using the anti-PD-Ll antibody, and the PD-L1 is visible as a first color (e.g., brown in the example shown in FIG. 1). Tumor cells with PD-L1 will show the first color (PD-L1), as indicated by the "+" sign in Column 4 of Table 1 (note that the tumor cells without PD-L1 do not have the first color, as indicated by the "-" sign in Column 4 of Table 1). Immune cells also exhibit PD-L1 expression and can be shown as the first color (PD-L1), as indicated by the "+" sign in Column 1 of Table 1. To differentiate between the two cell types that are positive for PD-L1, the sample is stained for a tumor-specific marker (e.g., cytokeratins detected by the pan keratin antibody), which is visible as a second color (a color different from the first color). The sample may also be stained for an immune cell-specific marker (e.g., CD4 or other marker), which is visible as a third color (a color different from the first and second colors).

**TABLE 1 (see summary below)**

|  | Column 1 | Column 2 | Column 3 | Column 4 |
|---|---|---|---|---|
|  | **Immune Cell with PD-L1** | **Immune Cell without PD-L1** | **Tumor Cell with PD-L1** | **Tumor Cell Without PD-L1** |
| **PD-L1 (first color)** | + | - | + | - |
| **Tumor Cell-Specific Differentiating Marker (second color)** | - | - | + | + |
| **Immune Cell-Specific Differentiating Marker (third color)** | + | + | - | - |

**Table 1 Summary:** Cells that have both the first color (PD-L1) and the second color (tumor cell-specific differentiating marker) but not the third color (immune cell-specific differentiating marker) are PD-L1 positive tumor cells (Column 3); cells that have both the first color (PD-L1) and third color (immune cell-specific differentiating marker) but not the second color are PD-L1 positive immune cells (Column 1); and cells that have the second color (tumor cell-specific differentiating marker) but not the first color (PD-L1) nor the third color (immune cell-specific differentiating marker) are PD-L1 negative tumor cells (Column 4). Cells that have the third color but not the first color and second color are PD-L1 negative immune cells (Column 2). The present invention is not limited to staining in any particular order. For example, Example 1 describes staining first for PD-L1, then staining for the tumor cell-specific marker, then staining for the immune cell-specific marker, and finally using a counterstain. However, in some embodiments, the order of the staining is different. For example, in some embodiments, the immune cell-specific marker is stained before the tumor cell-specific marker is stained, etc.

As previously described, the assays of the present invention feature staining of PD-L1 as well as staining of one or more differentiating markers. Methods of staining may include immunohistochemisty (IHC). Staining techniques may be performed on various biological samples, such as tissue (e.g., fresh frozen, formalin-fixed paraffin-embedded (FFPE)) and cytological samples. Chromogenic labels are used for recognition of the markers or targets (e.g., PD-L1, tumor cell-specific marker, immune cell-specific marker, etc.). Labels are well known to one of ordinary skill in the art and are not limited to the labels described herein.

A non-limiting example of a detailed protocol is described in Example 1 below. Briefly, samples of interest are stained for PD-L1. The sample is incubated first with an anti-PD-Ll primary antibody. The anti-PD-Ll primary antibody is detected with a first color. In Example 1, the sample is incubated with a horseradish peroxidase (HRP)-conjugated secondary antibody against the primary anti-PD-Ll antibody and a substrate (3,3'-diaminobenzidine (DAB)) is added, producing the first color (e.g., brown). Alternative enzymes and substrates (and resulting colors are described below).

The sample is then stained for a first differentiating marker, e.g., a marker that is tumor cell-specific. Examples of tumor cell-specific biomarkers may include but are not limited to: cytokeratins detectable with the pan keratin antibody (e.g., basic cytokeratins, many of the acidic cytokeratins), other cytokeratins such as cytokeratin 7 (CK7) and cytokeratin 20 (CK20), chromogranin, synaptophysin, CD56, thyroid transcription factor-1 (TTF-1), p53, leukocyte common antigen (LCA), vimentin, smooth muscle actin, or the like (e.g., see Capelozzi, V., J Bras Pneumol. 2009;35(4):375-382). One of ordinary skill in the art can substitute another appropriate tumor cell-specific biomarker for the cytokeratins as described in Example 1. The sample is incubated first with a primary antibody (e.g., anti-pan keratin antibody) against the first differentiating marker (tumor cell-specific marker). The anti-differentiating marker primary antibody is detected with a second color. In Example, 1, the sample is incubated with a haptenized antibody against the anti-differentiating marker primary antibody, and then the sample is incubated with an alkaline phosphatase (AP)-conjugated anti-hapten antibody. The substrate Fast Red Chromogen produces the second color (e.g., red).

The sample may then be stained for an immune cell-specific marker. Non-limiting examples of immune cell-specific biomarkers include CD4 or any other CD marker. Immune cell-specific biomarkers are well known to one of ordinary skill in the art. One of ordinary skill in the art could substitute another appropriate immune cell-specific biomarker for CD4 as described in Example 1. The sample is incubated first with a primary antibody (e.g., anti-CD4 antibody) against the second differentiating marker (immune cell-specific marker). The anti-differentiating marker primary antibody is detected with a third color. In Example 1, the sample is incubated with a HRP-conjugated secondary antibody against the primary anti-differentiating marker antibody and the substrate HRP-Green Chromogen is added, producing the third color (e.g., green/blue).

In some embodiments, the samples are then counterstained, producing a fourth color (the fourth color being different from the first, second, and third colors). In some embodiments, the counterstain comprises hematoxyline; however, the counterstain is not limited to hematoxyline. Alternative counterstains are well known to one of ordinary skill in the art. For example, the counterstain may comprise methylene blue, nuclear red, toluidine blue, eosin, methyl green, or the like. The particular counterstain is generally selected to produce contrast so as to enhance visibility.

Following the staining procedure, the samples are then interpreted and scored (scoring is described below). In some embodiments, the results of the staining may be interpreted as described in Table 1. In the case of an assay that stains for PD-L1, a tumor cell-specific marker (e.g., cytokeratins), and an immune cell-specific marker, the cells that score for the first color and third color but not the second color are PD-L1 positive immune cells, cells that score for both the first color and the second color (but not the third color) are PD-L1 positive cancer cells, and cells that score for the second color but not the first color nor the third color are PD-L1 negative cancer cells. In some embodiments, the first color and second color overlap (or others overlap), producing a fifth (different) color. This overlap color may help scoring.

In some embodiments, the staining (e.g., PD-L1 and the differentiating marker) occurs sequentially. In some embodiments, the staining occurs simultaneously.

The present invention is not limited to staining in any particular order. For example, Example 1 describes staining first for PD-L1, then staining for the tumor cell-specific marker, then staining for the immune cell-specific marker, and finally using a counterstain. However, in some examples, the tumor cell-specific marker (or immune cell-specific marker) is stained first, followed by the PD-L1 staining, etc.

### SIGNALING CONJUGATES (IHC/ISH CHROMOGENIC SUBSTRATES)

The present invention is not limited to the signaling conjugates (e.g., enzymes and chromogenic substrates) used in Example 1 nor to the other signaling conjugates described herein. Alternative enzyme-chromogenic substrate pairs for detection methods (e.g., immunohistochemistry, etc.) are well known to one of ordinary skill in the art.

Traditionally, chromogenic substrates precipitate when activated by the appropriate enzyme. That is, the traditional chromogenic substance is converted from a soluble reagent into an insoluble, colored precipitate upon contacting the enzyme. Chromogenic substrates used for the present invention may be compatible with automated slide staining instruments and processes and/or automated detection and analysis instruments and software. This may enable high detection sensitivity and multiplexing capability.

For example, the enzyme of the secondary antibody comprises HRP, alkaline phosphatase (AP), glucose oxidase, beta-galactosidase, the like, and/or others described in WO Patent Application No. 20131484498. In some examples, the substrate comprises DAB, Fast Red and Fast Blue, Fast Red and Black (silver), nitro blue tetrazolium chloride (NBT), 5-bromo-4-chloro-3-indolyl phosphate (BCIP), x-gal, 3-amino-9-ethylcarbazole (AEC), 5-bromo-4-chloro-3-indolyl-beta-D-galactopyranoside (BCIG), p-nitrophenyl phosphate (PNPP), 2,2'-azinobis [3-ethylbenzothiazoline-6-sulfonic acid] (ABTS), 3,3',5,5'-tetramethylbenzidine (TMB), HRP-Green Chromogen, the like, and/or others described in WO Patent Application No. 20131484498. Example 2 (below) further describes alternative signaling conjugates.

The present invention is also not limited to any particular colors or color combinations. For example, in some embodiments, the first color (PD-L1) is brown; however, in some examples, the first color (PD-L1) may be any other appropriate color, e.g., red, blue, yellow, green, etc., depending on the enzyme-substrate combination. In some embodiments, the second color (e.g., cytokeratins, other markers) may be red/pink; however, in some examples, the second color (e.g., cytokeratins, other markers) may be any other appropriate color, e.g., brown, blue, yellow, green, etc., depending on the enzyme-substrate combination. In some embodiments, the third color (e.g., CD4, other markers) may be green/blue; however, in some examples, the third color (e.g., CD4, other markers) may be any other appropriate color, e.g., brown, red/pink, yellow, etc., depending on the enzyme-substrate combination. In some examples, the fourth color (counterstain) may be blue; however, the fourth color (counterstain) may be any other appropriate color, e.g., red, green, etc. In some examples, the fifth color (overlap of the two colors) may be purple (e.g., if the two colors are red and blue), green (e.g., if the two colors are yellow and blue), orange (e.g., if the two colors are red and yellow), etc., depending on the combination of the two colors.

### SCORING

Samples are then scored. The second color (and third color) directs scoring of PD-L1 in either the tumor cells

8

or the immune cells. The use of the third color may improve scoring. For example, the use of the third color may help to clarify which cell type (tumor vs. immune) is PD-L1 positive. This can help the accuracy of the calculation of the number of PD-L1 positive immune cells, PD-L1 negative tumor cells, and PD-L1 positive tumor cells.

A positive result (e.g., a "PD-L1 positive result) may be calculated in a variety of ways and is not limited to the examples described herein.

In some examples, the number of PD-L1 positive tumor cells or the area of PD-L1 that is associated with tumor cells (e.g., the area of the slide covered with PD-L1 due to tumor cells) or the percentage of PD-L1 positive tumor cells may be calculated and then factored into an equation to calculate and H score. For example, if the H score is above a threshold for PD-L1 positivity then the sample is PD-L1 positive, and if the H score is below the threshold for PD-L1 positivity then the sample is PD-L1 negative.

Non-limiting examples of scoring calculations are presented in Example 3. Some examples are briefly described below:

In some examples, a positive result is determined by calculating the percentage of PD-L1 positive tumor cells and determining if that percentage is above the threshold for positivity or a predetermined cut-off. For example, in some embodiments, the minimum percentage of PD-L1 cells that confers PD-L1 positivity, e.g., 5% or more PD-L1 positive tumor cells confers PD-L1 positivity, 10% or more PD-L1 positive tumor cells confers PD-L1 positivity, 25% or more PD-L1 positive tumor cells confers PD-L1 positivity, 50% or more PD-L1 positive tumor cells confers PD-L1 positivity, etc.

In some examples, a positive result is determined by calculating the number of PD-L1 positive tumor cells divided by the total number of cells (e.g., number of tumor plus immune cells) and determining if that value is above the threshold for positivity (e.g., value greater than 0.15, value greater than 0.25, value greater than 0.5, etc.).

In some examples, a positive result is determined by calculating the sum of the percentage of PD-L1 positive tumor cells and PD-L1 positive immune cells and determining if that value is above the threshold for positivity (e.g., value greater than 40, value greater than 50, value greater than 60, etc.).

In some examples, a positive result is determined by calculating the number of PD-L1 positive tumor cells divided by the number of PD-L1 negative tumor cells and determining if that value is above the threshold for positivity (e.g., value greater than 1.5, value greater than 1.8, value greater than 2, etc.).

In some examples, a positive result is determined by calculating the number of PD-L1 positive tumor cells divided by the sum of the number of PD-L1 negative tumor cells and the number of PD-L1 negative immune cells and determining if that value is above the threshold for positivity (e.g., greater than 1.1, greater than 1.3, etc.).

In some examples, a positive result is determined by calculating the number of PD-L1 positive tumor cells divided by the sum of the number of PD-L1 negative tumor cells and the number of PD-L1 positive immune cells and determining if that value is above the threshold for positivity (e.g., greater than 1.1, greater than 1.3, etc.).

In some examples, a positive result is determined by calculating the number of PD-L1 positive tumor cells divided by the number of PD-L1 positive immune cells and determining if that value is above the threshold for positivity. In some embodiments, a positive result is determined by calculating the number of PD-L1 positive tumor cells divided by the number of PD-L1 negative immune cells and determining if that value is above the threshold for positivity.

As discussed above, PD-L1 positivity may be determined by calculating the percentage of PD-L1 positive tumor cells. For example, staining in greater than about 1% of cells (e.g., tumor cells) is scored as PD-L1 positive. In another example, staining in greater than about 5% of cells (e.g., tumor cells) is scored as PD-L1 positive. In some embodiments, staining in greater than about 10% of cells (e.g., tumor cells) is scored as PD-L1 positive. In some examples, staining in greater than about 15% of cells (e.g., tumor cells) is scored as PD-L1 positive. In some examples, staining in greater than about 20% of cells (e.g., tumor cells) is scored as PD-L1 positive. In some examples, staining in greater than about 25% of cells (e.g., tumor cells) is scored as PD-L1 positive. In some examples, staining in greater than about 30% of cells (e.g., tumor cells) is scored as PD-L1 positive. In some examples, staining in greater than about 35% of cells (e.g., tumor cells) is scored as PD-L1 positive. In some examples, staining in greater than about 40% of cells (e.g., tumor cells) is scored as PD-L1 positive. In some examples, staining in greater than about 45% of cells (e.g., tumor cells) is scored as PD-L1 positive. In some embodiments, staining in greater than about 50% of cells (e.g., tumor cells) is scored as PD-L1 positive. In some examples, staining in greater than about 55% of cells (e.g., tumor cells) is scored as PD-L1 positive. In some examples, staining in greater than about 60% of cells (e.g., tumor cells) is scored as PD-L1 positive. In some examples, staining in greater than about 65% of cells (e.g., tumor cells) is scored as PD-L1 positive. In some examples, staining in greater than about 70% of cells (e.g., tumor cells) is scored as PD-L1 positive. In some examples, staining in greater than about 75% of cells (e.g., tumor cells) is scored as PD-L1 positive. In some examples, staining in greater than about 80% of cells (e.g., tumor cells) is scored as PD-L1 positive. In some examples, staining in greater than about 90% of cells (e.g., tumor cells) is scored as PD-L1 positive.

The number or percentage of PD-L1 positive immune cells may be relevant to the determination of PD-L1 positivity. For example, staining in greater than about 5% of immune cells is associated with scoring as PD-L1 positive. In some embodiments, staining in greater than about 10% of immune cells is associated with scoring as PD-L1 positive. In some examples, staining in greater than about 15% of immune cells is associated with scoring as PD-L1 positive. In some examples, staining in greater than about 25% of immune cells is associated with scoring as PD-L1 positive. In some embodiments, staining in greater than about 50% of immune cells is associated with scoring as PD-L1 positive.

The positivity of the sample may also be determined by the degree or intensity of staining (e.g., heavy staining may be positive and light staining may be negative). In some embodiments, scoring methods may feature scoring samples on an intensity scale, e.g., of 0 to 3, for PD-L1 expression (see for example, U.S. Provisional Patent Application No. 61/875,334 (Scoring Method For Mesothelin Protein Expression). In some embodiments, samples are scored based on intensity and percentages of cells staining. For example, as described in U.S. Provisional Patent Application No. 61/875334, H scores are calculated as: 1 * (percentage of tumor cells staining at 1+ intensity) + 2 * (percentage of tumor cells staining at 2+ intensity) + 3 * (percentage of cells staining at 3+ intensity) = H score (a value between 0 and 300). Other scoring methods have been described and are well known to one of ordinary skill in the art.

## EXAMPLE 1 - PROTOCOL FOR MULTIPLEX ASSAY

Example 1 describes a non-limiting example of a multiplex IHC assay of the present invention. A NSCLC sample slide is prepared according to standard protocols.

1. Apply 1 drop of PD-L1 SP142 antibody (Ventana Medical System, Tucson, Arizona) to the slide and incubate for 16 minutes. Rinse slide with reaction buffer.

2. Apply 1 drop of OptiView HQ Universal Linker (Catalog No. 760-700, Ventana Medical Systems, Tucson, Arizona) and incubate for 8 minutes. Rinse slide with Reaction Buffer.

3. Apply 1 drop of OptiView HRP Multimer (Catalog No. 760-700, Ventana Medical System, Tucson, Arizona) and incubate for 8 minutes. Rinse slide with Reaction Buffer.

4. Apply 1 drop each of OptiView Amplifier $H_2O_2$ and OptiView Amplifier (Catalog No. 760-700, Ventana Medical System, Tucson, Arizona) and incubate for 8 minutes. Rinse slide with Reaction Buffer.

5. Apply 1 drop of OptiView Amplifier Multimer (Catalog No. 760-700, Ventana Medical System, Tucson, Arizona) and incubate for 8 minutes. Rinse slide with Reaction Buffer.

6. Apply 1 drop of OptiView $H_2O_2$ and 1 drop of OptiView DAB (Catalog No. 760-700, Ventana Medical Systems, Tucson, Arizona) and incubate for 8 minutes. Rinse slide with Reaction Buffer.

7. Apply 1 drop of OptiView Copper (Catalog No. 760-700, Ventana Medical Systems, Tucson, Arizona) and incubate for 4 minutes. Rinse slide with Reaction Buffer.

8. Apply 1 drop of Pan Keratin Antibody (AE1/AE3/PCK26) Primary Antibody (Catalog No. 760-2595, Ventana Medical Systems, Tucson, Arizona). Incubate for 8 minutes. Rinse slide with Reaction Buffer.

9. Apply 1 drop of Haptenized anti-mouse antibody and incubate for 8 minutes. Rinse slide with reaction buffer.

10. Apply 1 drop of AP-conjugated anti-hapten antibody and incubate for 8 minutes. Rinse slide with Reaction Buffer.

11. Apply Fast Red chromogen and incubate for 8 minutes. Rinse with Reaction Buffer.

12. Apply 1 drop of anti-CD4 (SP35) rabbit monoclonal primary antibody (Catalog No. 790-4423, Ventana Medical Systems, Tucson, Arizona) and incubate for 16 minutes. Rinse slide with Reaction Buffer.

13. Apply 1 drop of HRP-conjugated anti-rabbit antibody and incubate for 16 minutes. Rinse slide with Reaction Buffer.

14. Apply 2 drops of HRP-Green Chromogen Detection 1 and incubate for 4 minutes.

15. Apply 2 drops of HRP-Green Chromogen Detection 2 and incubate for 12 minutes. Rinse slide with Reaction Buffer.

16. Apply 1 drop of Mayer's Hematoxyline (1:5) and incubate for 4 minutes. Rinse slide with Reaction Buffer.

**EXAMPLE 2 - SIGNALING CONJUGATES**

The following example describes alternative signaling conjugates described in WO Patent Application No. 2013148498.

In some embodiments, methods of detecting a target in a biological sample include contacting the biological sample with a detection probe, contacting the biological sample with a labeling conjugate, and contacting the biological sample with a signaling conjugate. The labeling conjugate includes an enzyme. The signaling conjugate includes a latent reactive moiety and a chromogenic moiety. The enzyme catalyzes conversion of the latent reactive moiety into a reactive moiety, which covalently binds to the biological sample proximally to or directly on the target. The method further includes illuminating the biological sample with light and detecting the target through absorbance of the light by the chromogenic moiety of the signaling conjugate. In one embodiment, the reactive moiety reacts with a tyrosine residue of the biological sample, the enzyme conjugate, the detection probe, or combinations thereof.

In some embodiments, the detection probe is an antibody probe. In some embodiments, the labeling conjugate includes an antibody coupled to the enzyme. Enzymes may include oxidoreductases, peroxidases, or hydrolases. An antibody for the labeling conjugate may be an anti-species or an anti-hapten antibody. The detection probe may include a hapten selected from the group consisting an oxazole hapten, pyrazole hapten, thiazole hapten, nitroaryl hapten, benzofuran hapten, triterpene hapten, urea hapten, thiourea hapten, rotenoid hapten, coumarin hapten, cyclolignan hapten, di-nitrophenyl hapten, biotin hapten, digoxigenin hapten, fluorescein hapten, and rhodamine hapten. In other examples, the detection probe is monoclonal antibody derived from a second species such as goat, rabbit, mouse, or the like. The labeling conjugate is configured, through its inclusion of an anti-species or an anti-hapten antibody to bind selectively to the detection probe.

Chromogen conjugates used for the present invention may be configured to absorb light more selectively than traditionally available chromogens. Detection is realized by absorbance of the light by the signaling conjugate; for example, absorbance of at least about 5% of incident light would facilitate detection of the target. In other darker stains, at least about 20% of incident light would be absorbed. Non-uniform absorbance of light within the visible spectra results in the chromophore moiety appearing colored. The signaling conjugates disclosed herein may appear colored due to their absorbance; the signaling conjugates may appear to provide any color when used in the assay, with certain particular colors including red, orange, yellow, green, indigo, or violet depending on the spectral absorbance associated with the chomophore moiety. According to another aspect, the chromophore moieties may have narrower spectral absorbances than those absorbances of traditionally used chromogens (e.g. DAB, Fast Red, Fast Blue). In illustrative embodiments, the spectral absorbance associated with the first chromophore moiety of the first signaling conjugate has a full-width half-max (FWHM) of between about 30 nm and about 250 nm, between about 30 nm and about 150 nm, between about 30 nm and about 100 nm, or between about 20 nm and about 60 nm.

Narrow spectral absorbances enable the signaling conjugate chromophore moiety to be analyzed differently than traditional chromogens. While having enhanced features compared to traditionally chromogens, detecting the signaling conjugates remains simple. In illustrative embodiments, detecting comprises using a bright-field microscope or an equivalent digital scanner. The narrow spectral absorbances enable chromogenic multi-plexing at level beyond the capability of traditional chromogens. For example, traditional chromogens are somewhat routinely duplexed (e.g. Fast Red and Fast Blue, Fast Red and Black (silver), Fast Red and DAB). However, triplexed or three-color applications, or greater, are atypical, as it becomes difficult to discern one chromophore from another. In illustrative embodiments of the presently disclosed technology, the method includes detecting from two to at least about six different targets using different signaling conjugates or combinations thereof. In one embodiment, illuminating the biological sample with light comprises illuminating the biological sample with a spectrally narrow light source, the spectrally narrow light source having a spectral emission with a second full-width half-max (FWHM) of between about 30 nm and about 250 nm, between about 30 nm and about 150 nm, between about 30 nm and about 100 nm, or between about 20 nm and about 60 nm. In another embodiment, illuminating the biological sample with light includes illuminating the biological sample with an LED light source. In another embodiment, illuminating the biological sample with light includes illuminating the biological sample with a filtered light source.

In illustrative embodiments, detecting targets within the sample includes contacting the biological sample with a first amplifying conjugate that is covalently deposited proximally to or directly on the first labeling conjugate. The first amplifying conjugate may be followed by contacting the biological sample with a secondary labeling conjugate. Illustratively, the amplification of signal using amplifying conjugates enhances the deposition of signaling conjugate. The enhanced deposition of signaling conjugate enables easier visual identification of the chromogenic signal, that is, the amplification makes the color darker and easier to see. For low expressing targets, this amplification may result in the signal becoming sufficiently dark to be visible, whereas without amplification, the target would not be apparent. In one embodiment, the signaling conjugate is covalently deposited proximally to the target at a concentration of greater than about $1 \times 10^{11}$ molecules per $cm^{2}*\mu m$ to about $1 \times 10^{16}$ molecules per $cm^{2}*\mu m$ of the biological sample. In one embodiment, the first target and the second target are genetic nucleic acids. Detecting the first target through absorbance of the light by the first signaling conjugate includes detecting a first colored signal selected from red, orange, yellow, green, indigo, or violet, the first colored signal associated with spectral absorbance associated with the first chromogenic moiety of the first signaling conjugate. Detecting the second target through absorbance of the light by the second signaling conjugate includes detecting a second colored signal selected from red, orange, yellow, green, indigo, or violet, the second colored signal associated with spectral absorbance associated with the second chromogenic moiety of the second signaling conjugate. Detecting an overlap in proximity through absorbance of the light by the first signaling conjugate overlapping in proximity with the second signaling conjugate so that a third colored signal associated with overlapping spectral absorbance of the first spectral absorbance and the second spectral absorbance. According to one example, this third color signals a normal genetic arrangement and the first and second colors signal a genetic rearrangement or translocation.

**EXAMPLE 3 - SCORING**

The following example describes various calculations (3A-3E) for determining PD-L1 positivity.

*Example 3A*

$$\text{Equation: } \textbf{PD-L1 Value = Percentage of PD-L1 positive tumor cells}$$

Threshold for positivity: **PD-L1 Value > 40% is PD-L1 positive**

A pathologist views Sample 3A and calculates the percentage of PD-L1 positive tumor cells as being 48%. Based on the threshold for positivity, Sample 3A is labeled PD-L1 positive.

*Example 3B*

$$\text{Equation: } \textbf{PD-L1 Value = \# of PD-L1 positive tumor cells/ total \# of cells}$$

Threshold for positivity: **PD-L1 Value > 0.25 is PD-L1 positive**

A pathologist views Sample 3B. The number of PD-L1 positive tumor cells is 68, and the total number of cells is 460. The PD-L1 value based on the above calculation is 68/460 = 0.147. Based on the threshold for positivity, Sample 3B is labeled PD-L1 negative.

*Example 3C*

$$\text{Equation: } \textbf{PD-L1 Value = \% of PD-L1 positive tumor cells + \% PD-L1 positive immune cells}$$

Threshold for positivity: **PD-L1 Value > 60 is PD-L1 positive**

A pathologist views Sample 3C. The percent of PD-L1 positive tumor cells is 50, and the percent of PD-L1 positive immune cells is 20. The PD-L1 value based on the above calculation is 50 + 20 = 70. Based on the threshold for positivity, Sample 3C is labeled PD-L1 positive.

*Example 3D*

$$\text{Equation: } \textbf{PD-L1 Value} = \text{\# of PD-L1 positive tumor cells/ (\# of PD-L1 negative tumor cells} + \text{\# of PD-L1 positive immune cells)}$$

Threshold for positivity: **PD-L1 Value > 0.8 is PD-L1 positive**

A pathologist views Sample 3D. The number of PD-L1 positive tumor cells is 68, the number of PD-L1 negative tumor cells is 45, and the number of PD-L1 positive immune cells is 210. The PD-L1 value based on the above calculation is 68/(45+210) = 0.266. Based on the threshold for positivity, Sample 3D is labeled PD-L1 negative.

*Example 3E*

$$\text{Equation: } \textbf{H score} = 1 * \text{(percentage of tumor cells staining at 1+ intensity)} + 2 * \text{(percentage of tumor cells staining at 2+ intensity)} + 3 * \text{(percentage of cells staining at 3+ intensity)}$$

Threshold for positivity: **H score > 125 is PD-L1 positive**

A pathologist views Sample 3E. The percentage of PD-L1 positive tumor cells staining at 1+ intensity is 5%, the percentage of PD-L1 positive tumor cells staining at 2+ intensity is 35%, and the percentage of PD-L1 positive tumor cells staining at 3+ intensity is 20%. The H score is 5(1) + 2(35) + 3 (20) = 135. Based on the threshold for positivity, Sample 3E is labeled PD-L1 positive.
As used herein, the term "about" refers to plus or minus 10% of the referenced number.
Various modifications of the invention, in addition to those described herein, will be apparent to those skilled in the art from the foregoing description. Such modifications are also intended to fall within the scope of the appended claims. For example, an "antibody" used in accordance with the present invention may be a whole antibody or a fragment of an antibody that is effective in binding to a desired target site. Also, when appropriate, an "antibody" of the present invention may be substituted with a targeting moiety (e.g., ligand peptide, small molecule, etc.). For example, if the tumor cell or the immune cell has a specific, differentiating and unique cell surface receptor, then a corresponding targeting moiety may be used in accordance with the present invention to differentiate tumor cells from immune cells.

**Claims**

1. A method of scoring PD-L1 expression in a tumor sample, the method comprising:

    • labeling the tumor tissue sample, the labeling comprising:

        ◦ contacting the tissue sample with an anti-PD-Ll primary antibody; and
        ◦ contacting the same tissue sample with
        ◦ a primary antibody directed to a tumor cell-specific marker and an antibody directed to an immune cell-specific marker; and
        ◦ visualizing each of the antibodies in the tissue sample with a reagent that generates a detectable signal corresponding to each of the primary antibodies, wherein the anti-PD-Ll antibody has a first detectable signal, the antibody directed to the tumor cell-specific marker has a second detectable signal distinguishable from the first detectable signal, and the antibody directed to an immune cell-specific marker has a third detectable signal distinguishable from the first detectable signal and the second detectable signal;

    wherein the first, second, and third detectable signals are generated by chromogens; and

• scoring PD-L1 expression in tumor cells, immune cells, or both, wherein co-localization of the first and second detectable signals indicates the presence of PD-L1-positive tumor cells and co-localization of the first and third detectable signals indicates the presence of PD-L1-positive immune cells.

2. The method of claim 1, wherein the tumor cell-specific marker is selected from the group consisting of a cytokeratin, chromogranin, synaptophysin, CD56, thyroid transcription factor-1 (TTF-1), p53, leukocyte common antigen (LCA), vimentin, and smooth muscle actin, and wherein the immune cell-specific marker is selected from the group consisting of CD3, CD4, CD8, CD19, CD20, CD11c, CD123, CD56, CD14, CD33, or CD66b.

3. The method of claim 1 or 2, wherein the immune cell-specific marker is a T-cell marker or a B-cell marker.

4. The method of claim 1, wherein the antibody directed to the tumor cell-specific marker is a pan-keratin antibody and the antibody directed to the immune cell-specific marker is an anti-CD4 antibody.

5. The method of any of claims 1 to 4, wherein:

• the first detectable signal is generated by

∘ contacting the tissue sample with a horseradish peroxidase (HRP)-conjugated secondary antibody that recognizes the anti-PD-L1 primary antibody;
∘ reacting the HRP with 3,3'-Diaminobenzidine (DAB) to produce a brown color;

• the second detectable signal is generated by:

∘ contacting the sample with an alkaline phosphatase (AP) labeled antibody that recognizes the primary antibody directed to a tumor cell-specific marker;
∘ reacting the AP with a Fast Red chromogen and naphthol to produce a red color; and

• the third detectable signal is generated by

∘ contacting the sample with a HRP-conjugated secondary antibody that recognizes the primary antibody directed to an immune cell-specific marker;
∘ reacting the HRP with HRP-green chromogen to produce a green color.

6. The method of any of claims 1 to 5, wherein the first, second, and/or third detectable signal is an amplified signal, optionally wherein the amplified signal is generated by tyramide signal amplification.

7. The method of any of claims 1 to 6, wherein contacting the sample with the primary antibodies is performed simultaneously, or wherein contacting the sample with the primary antibodies is performed sequentially.

8. The method of any of claims 1 to 7, further comprising counterstaining the tissue sample, the counterstain producing a fourth detectable signal that is distinguishable from the first, second, and the third detectable signals, optionally wherein the counterstain comprises hematoxylin.

9. The method of any of claims 1 to 8, wherein a fifth detectable signal is produced by overlap of the first detectable signal and the second detectable signal.

10. The method of claim 9, wherein a sixth detectable signal is produced by overlap of the first detectable signal and the third detectable signal.

11. The method of any of claims 1 to 10, wherein the total number of PD-L1 positive and PD-L1-negative tumor cells is quantitated, and wherein the tumor is scored as PD-L1 positive if staining for PD-L1 is detected in greater than about 10% of tumor cells, or the tumor is scored as PD-L1 positive if staining for PD-L1 is detected in greater than about 50% of tumor cells.

12. The method of any of claims 1 to 10, wherein the total number of PD-L1 positive and PD-L1-negative immune cells is quantitated, and wherein the tumor is scored as PD-L1 positive if staining for PD-L1 is detected in greater than about 10% of immune cells, or the tumor is scored as PD-L1 positive if staining for PD-L1 is detected in greater than about 50% of immune cells.

13. The method of claims 1 to 10, wherein PD-L1-positive immune cells, PD-L1 positive tumor cells, and PD-L1 negative tumor cells are quantitated to generate a PD-L1 Value, wherein:

$$\text{PD-L1 Value} = \text{PD-L1 positive tumor cells} / (\text{PD-L1 negative tumor cells} + \text{PD-L1 positive immune cells}),$$

wherein:

• **PD-L1 positive tumor cells** is calculated either by counting the number of cells staining for both the first and second detectable signals or by calculating the area of the tissue sample in which the first detectable signal is associated with the second detectable signal;
• **PD-L1 negative tumor cells** is calculated either by counting the number of cells staining for the second detectable signal only or by calculating the area of the tissue sample in which the second detectable signal is not associated with the first detectable signal; and
• **PD-L1 positive immune cells** is calculated either by counting the number of cells staining for both the first and third detectable signals or by calculating the area of the tissue sample in which the first detectable signal is associated with the third detectable signal.

14. The method of any of claims 1 to 10, further comprising scoring intensity of PD-L1 staining in PD-L1 positive tumor cells and calculating an H score, wherein:

$$\text{H score} = 1 * (\text{percentage of PD-L1 positive tumor cells staining at 1+ intensity}) + 2 * (\text{percentage of PD-L1 positive tumor cells staining at 2+ intensity}) + 3 * (\text{percentage of PD-L1 positive tumor cells staining at 3+ intensity}).$$

**Patentansprüche**

1. Verfahren zur Bewertung der PD-L1-Expression in einer Tumorprobe, wobei das Verfahren Folgendes umfasst:

• Markieren der Tumorgewebeprobe, wobei das Markieren Folgendes umfasst:

◦ Inkontaktbringen der Gewebeprobe mit einem Anti-PD-Ll-Primärantikörper; und
◦ Inkontaktbringen der gleichen Gewebeprobe mit
◦ einem Primärantikörper, der auf einen tumorzellenspezifischen Marker gerichtet ist, und einem Antikörper, der auf einen immunzellenspezifischen Marker gerichtet ist; und
◦ Visualisieren jedes der Antikörper in der Gewebeprobe mit einem Reagenz, das ein nachweisbares Signal erzeugt, entsprechend jedem der Primärantikörper, wobei der Anti-PD-Ll-Antikörper ein erstes nachweisbares Signal aufweist, der Antikörper, der auf den tumorzellenspezifischen Marker gerichtet ist, ein zweites nachweisbares Signal aufweist, das von dem ersten nachweisbaren Signal unterscheidbar ist, und der Antikörper, der auf einen immunzellenspezifischen Marker gerichtet ist, ein drittes nachweisbares Signal aufweist, das von dem ersten nachweisbaren Signal und dem zweiten nachweisbaren Signal unterscheidbar ist;

wobei das erste, zweite und dritte nachweisbare Signal durch Chromogene erzeugt werden; und
• Bewertung der PD-Ll-Expression in Tumorzellen, Immunzellen oder beiden, wobei die Kolokalisation des ersten und zweiten nachweisbaren Signals auf die Anwesenheit von PD-L1-positiven Tumorzellen hinweist und die Kolokalisation des ersten und dritten nachweisbaren Signals auf die Anwesenheit von PD-L1 -positiven Immunzellen hinweist.

2. Verfahren nach Anspruch 1, wobei der tumorzellenspezifische Marker ausgewählt ist aus der Gruppe bestehend aus einem Zytokeratin, Chromogranin, Synaptophysin, CD56, Schilddrüsentranskriptionsfaktor-1 (TTF-1), p53, gemeinsamen Leukozytenantigen (LCA), Vimentin, und Glattmuskelaktin, und wobei der immunzellenspezifische Marker ausgewählt ist aus der Gruppe bestehend aus CD3, CD4, CD8, CD19, CD20, CD11c, CD123, CD56, CD14, CD33 oder CD66b.

3. Verfahren nach Anspruch 1 oder 2, wobei der immunzellenspezifische Marker ein T-Zell-Marker oder ein B-Zell-Marker ist.

4. Verfahren nach Anspruch 1, wobei der Antikörper, der auf den tumorzellenspezifischen Marker gerichtet ist, ein Pankeratin-Antikörper ist und der Antikörper, der auf den immunzellenspezifischen Marker gerichtet ist, ein Anti-CD4-Antikörper ist.

5. Verfahren nach einem der Ansprüche 1 bis 4, wobei:

   • das erste nachweisbare Signal erzeugt wird durch

   ◦ Inkontaktbringen der Gewebeprobe mit einem mit Meerrettichperoxidase konjugierten (HRP-konjugierten) Sekundärantikörper, der den Anti-PD-Ll-Primärantikörper erkennt;
   ◦ Reagieren der HRP mit 3,3 '-Diaminobenzidin (DAB), um eine braune Farbe zu erzeugen;

   • das zweite nachweisbare Signal erzeugt wird durch:

   ◦ Inkontaktbringen der Probe mit einem mit alkalischer Phosphatase (AP) markierten Antikörper, der den Primärantikörper, der auf einen tumorzellenspezifischen Marker gerichtet ist, erkennt;
   ◦ Reagieren der AP mit einem Fast-Red-Chromogen und Naphthol, um eine rote Farbe zu erzeugen; und

   • das dritte nachweisbare Signal erzeugt wird durch

   ◦ Inkontaktbringen der Probe mit einem mit HRP-konjugierten Sekundärantikörper, der den Primärantikörper, der auf einen immunzellenspezifischen Marker gerichtet ist, erkennt;
   ◦ Reagieren der HRP mit HRP-Grün-Chromogen, um eine grüne Farbe zu erzeugen.

6. Verfahren nach einem der Ansprüche 1 bis 5, wobei das erste, zweite und/oder dritte nachweisbare Signal ein amplifiziertes Signal ist, gegebenenfalls wobei das amplifizierte Signal durch Tyramidsignalamplifikation erzeugt wird.

7. Verfahren nach einem der Ansprüche 1 bis 6, wobei das Inkontaktbringen der Probe mit den Primärantikörpern gleichzeitig durchgeführt wird, oder wobei das Inkontaktbringen der Probe mit den Primärantikörpern nacheinander durchgeführt wird.

8. Verfahren nach einem der Ansprüche 1 bis 7, ferner umfassend das Gegenfärben der Gewebeprobe, wobei die Gegenfärbung ein viertes nachweisbares Signal ergibt, das von dem ersten, zweiten und dem dritten nachweisbaren Signal unterscheidbar ist, gegebenenfalls wobei die Gegenfärbung Hämatoxylin umfasst.

9. Verfahren nach einem der Ansprüche 1 bis 8, wobei ein fünftes nachweisbares Signal durch Überlappen des ersten nachweisbaren Signals und des zweiten nachweisbaren Signals erzeugt wird.

10. Verfahren nach Anspruch 9, wobei ein sechstes nachweisbares Signal durch Überlappen des ersten nachweisbaren Signals und des dritten nachweisbaren Signals erzeugt wird.

11. Verfahren nach einem der Ansprüche 1 bis 10, wobei die Gesamtzahl der PD-L1-positiven und PD-L1-negativen Tumorzellen quantifiziert wird, und wobei der Tumor als PD-L1-positiv bewertet wird, wenn eine Färbung für PD-L1 bei mehr als etwa 10 % der Tumorzellen nachgewiesen wird, oder der Tumor als PD-L1-positiv bewertet wird, wenn eine Färbung für PD-L1 bei mehr als etwa 50 % der Tumorzellen nachgewiesen wird.

12. Verfahren nach einem der Ansprüche 1 bis 10, wobei die Gesamtzahl der PD-L1-positiven und PD-L1-negativen Immunzellen quantifiziert wird, und wobei der Tumor als PD-L1-positiv bewertet wird, wenn eine Färbung für PD-L1 bei mehr als etwa 10 % der Immunzellen nachgewiesen wird, oder der Tumor als PD-L1-positiv bewertet wird, wenn eine Färbung für PD-L1 bei mehr als etwa 50 % der Immunzellen nachgewiesen wird.

13. Verfahren nach den Ansprüchen 1 bis 10, wobei PD-L1-positive Immunzellen, PD-L1 positive Tumorzellen und PD-L1-negative Tumorzellen quantifiziert werden, um einen PD-L1-Wert zu erzeugen, wobei:

**PD-L1-Wert = PD-L1-positive Tumorzellen / (PD-L1-negative Tumorzellen + PD-L1-positive Immunzellen),**

wobei:

• **PD-L1-positive Tumorzellen** entweder durch Zählen der Anzahl an sowohl für das erste als auch für das zweite nachweisbare Signal gefärbten Zellen oder durch Berechnen der Oberfläche der Gewebeprobe, in der das erste nachweisbare Signal mit dem zweiten nachweisbaren Signal assoziiert ist, berechnet wird;
• **PD-L1-negative Tumorzellen** entweder durch Zählen der Anzahl an nur für das zweite nachweisbare Signal gefärbten Zellen oder durch Berechnen der Oberfläche der Gewebeprobe, in der das zweite nachweisbare Signal nicht mit dem ersten nachweisbaren Signal assoziiert ist, berechnet wird; und
• **PD-L1-positive Immunzellen** entweder durch Zählen der Anzahl an sowohl für das erste als auch für das dritte nachweisbare Signal gefärbten Zellen oder durch Berechnen der Oberfläche der Gewebeprobe, in der das erste nachweisbare Signal mit dem dritten nachweisbaren Signal assoziiert ist, berechnet wird.

14. Verfahren nach einem der Ansprüche 1 bis 10, ferner umfassend die Bewertung der Intensität der PD-L1-Färbung in PD-L1-positiven Tumorzellen und Berechnen eines H-Werts, wobei:

**H-Wert = 1 \* (Prozent an PD-L1-positiven Tumorzellen mit Färbung bei 1+ Intensität) + 2 \* (Prozent an PD-L1-positiven Tumorzellen mit Färbung bei 2+ Intensität) + 3 \* (Prozent an PD-L1-positiven Tumorzellen mit Färbung bei 3+ Intensität).**

**Revendications**

1. Procédé de détermination des scores de l'expression de PD-L1 dans un échantillon de tumeur, le procédé comprenant :

• le marquage de l'échantillon de tissu tumoral, le marquage comprenant :

◦ la mise en contact de l'échantillon de tissu avec un anticorps primaire anti-PD-L1 ; et
◦ la mise en contact de ce même échantillon de tissu avec
◦ un anticorps primaire dirigé contre un marqueur spécifique d'une cellule tumorale et un anticorps dirigé contre un marqueur spécifique d'une cellule immunitaire ; et
◦ la visualisation de chacun des anticorps dans l'échantillon de tissu avec un réactif qui génère un signal détectable correspondant à chacun des anticorps primaires, dans laquelle l'anticorps anti-PD-L1 a un premier signal détectable, l'anticorps dirigé contre le marqueur spécifique d'une cellule tumorale a un deuxième signal détectable distinguable du premier signal détectable, et l'anticorps dirigé contre un marqueur spécifique d'une cellule immunitaire a un troisième signal détectable distinguable du premier signal détectable et du deuxième signal détectable ;

dans lequel les premier, deuxième et troisième signaux détectables sont générés par des chromo gènes ; et
• la détermination des scores de l'expression de PD-L1 dans les cellules tumorales, les cellules immunitaires ou les deux, dans laquelle la co-localisation des premier et deuxième signaux détectables indique la présence de cellules tumorales positives à PD-L1 et la co-localisation des premier et troisième signaux détectables indique la présence de cellules immunitaires positives à PD-L1.

**2.** Procédé selon la revendication 1, dans lequel le marqueur spécifique d'une cellule tumorale est choisi dans le groupe constitué par une cytokératine, une chromogranine, une synaptophysine, le CD56, le facteur de transcription thyroïdien-1 (TTF-1), le p53, un antigène leucocytaire commun (LCA), la vimentine, et l'actine des muscles lisses, et dans lequel le marqueur spécifique d'une cellule immunitaire est choisi dans le groupe constitué par CD3, CD4, CD8, CD19, CD20, CD11c, CD123, CD56, CD14, CD33 ou CD66b.

**3.** Procédé selon la revendication 1 ou 2, dans lequel le marqueur spécifique d'une cellule immunitaire est un marqueur des lymphocytes T ou un marqueur des lymphocytes B.

**4.** Procédé selon la revendication 1, dans lequel l'anticorps dirigé contre le marqueur spécifique d'une cellule tumorale est un anticorps anti-pan-kératine et l'anticorps dirigé contre le marqueur spécifique d'une cellule immunitaire est un anticorps anti-CD4.

**5.** Procédé selon l'une quelconque des revendications 1 à 4, dans lequel :

• le premier signal détectable est généré par

◦ la mise en contact de l'échantillon de tissu avec un anticorps secondaire conjugué à la peroxydase de raifort (HPR) qui reconnait l'anticorps primaire anti-PD-L1 ;
◦ la mise en réaction de la HPR avec de la 3,3'-diaminobenzidine (DAB) pour produire une couleur marron ;

• le deuxième signal détectable est généré par :

◦ la mise en contact de l'échantillon avec un anticorps marqué par une phosphatase alcaline (PA) qui reconnait l'anticorps primaire dirigé contre un marqueur spécifique d'une cellule tumorale ;
◦ la mise en réaction de la PA avec un chromogène Fast Red et du naphtol pour produire une couleur rouge ; et

• le troisième signal détectable est généré par

◦ la mise en contact de l'échantillon avec un anticorps secondaire conjugué à la HPR qui reconnait l'anticorps primaire dirigé contre un marqueur spécifique d'une cellule immunitaire ;
◦ la mise en réaction de la HPR avec un chromogène HPR-vert pour produire une couleur verte.

**6.** Procédé selon l'une quelconque des revendications 1 à 5, dans lequel le premier, le deuxième et/ou le troisième signal détectable est un signal amplifié, éventuellement dans lequel le signal amplifié est généré par une amplification de signal de tyramide.

**7.** Procédé selon l'une quelconque des revendications 1 à 6, dans lequel la mise en contact de l'échantillon avec les anticorps primaires est réalisée simultanément, ou dans lequel la mise en contact de l'échantillon avec les anticorps primaires est réalisée successivement.

**8.** Procédé selon l'une quelconque des revendications 1 à 7, comprenant en outre la contre-coloration de l'échantillon de tissu, la contre-coloration produisant un quatrième signal détectable qui est distinguable des premier, deuxième et troisième signaux détectables, éventuellement dans lequel la contre-coloration comprend de l'hématoxyline.

**9.** Procédé selon l'une quelconque des revendications 1 à 8, dans lequel un cinquième signal détectable est produit par chevauchement du premier signal détectable et du deuxième signal détectable.

**10.** Procédé selon la revendication 9, dans lequel un sixième signal détectable est produit par chevauchement du premier signal détectable et du troisième signal détectable.

**11.** Procédé selon l'une quelconque des revendications 1 à 10, dans lequel le nombre total de cellules tumorales positives à PD-L1 et négatives à PD-L1 est quantifié, et dans lequel le score de la tumeur est déterminé comme positif à PD-L1 si une coloration pour PD-L1 est détectée dans plus d'environ 10 % des cellules tumorales, ou le score de la tumeur est déterminé comme positif à PD-L1 si une coloration pour PD-L1 est détectée dans plus d'environ 50 % des cellules tumorales.

**12.** Procédé selon l'une quelconque des revendications 1 à 10, dans lequel le nombre total de cellules immunitaires positives à PD-L1 et négatives à PD-L1 est quantifié, et dans lequel le score de la tumeur est déterminé comme positif à PD-L1 si une coloration pour PD-L1 est détectée dans plus d'environ 10 % des cellules immunitaires, ou le score de la tumeur est déterminé comme positif à PD-L1 si une coloration pour PD-L1 est détectée dans plus d'environ 50 % des cellules immunitaires.

**13.** Procédé selon les revendications 1 à 10, dans lequel les cellules immunitaires positives à PD-L1, les cellules tumorales positives à PD-L1 et les cellules tumorales négatives à PD-L1 sont quantifiées pour générer une valeur de PD-L1, dans lequel :

$$\text{Valeur de PD-L1} = \text{cellules tumorales positives à PD-L1} / (\text{cellules tumorales négatives à PD-L1} + \text{cellules immunitaires positives à PD-L1}),$$

dans laquelle :

- **cellules tumorales positives à PD-L1** est calculée soit en comptant le nombre de cellules colorées pour à la fois les premier et deuxième signaux détectables soit en calculant la surface de l'échantillon de tissu dans lequel le premier signal détectable est associé au deuxième signal détectable ;
- **cellules tumorales négatives à PD-L1** est calculée soit en comptant le nombre de cellules colorées uniquement pour le deuxième signal détectable soit en calculant la surface de l'échantillon de tissu dans lequel le deuxième signal détectable n'est pas associé au premier signal détectable ; et
- **cellules immunitaires positives à PD-L1** est calculée soit en comptant le nombre de cellules colorées pour à la fois les premier et troisième signaux détectables soit en calculant la surface de l'échantillon de tissu dans lequel le premier signal détectable est associé au troisième signal détectable.

**14.** Procédé selon l'une quelconque des revendications 1 à 10, comprenant en outre la détermination du score de l'intensité de coloration de PD-L1 dans des cellules tumorales positives à PD-L1 et le calcul d'un score H, dans lequel :

$$\text{Score H} = 1 * (\text{pourcentage de coloration des cellules tumorales positives à PD-L1 à l'intensité +1}) + 2 * (\text{pourcentage de coloration des cellules tumorales positives à PD-L1 à l'intensité +2}) + 3 * (\text{pourcentage de coloration des cellules tumorales positives à PD-L1 à l'intensité +3}).$$

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62004572 A
- WO 2013148498 A
- WO 20131484498 A
- US 61875334 A

**Non-patent literature cited in the description**

- **FREEMAN et al.** *J Exp Med,* 2000, vol. 192, 1027-34
- **LATCHMAN et al.** *Nat Immunol,* 2001, vol. 2, 261-8
- **CARTER et al.** *Eur J Immunol,* 2002, vol. 32, 634-43
- **OHIGASHI et al.** *Clin Cancer Res,* 2005, vol. 11, 2947-53
- **DONG et al.** *Nat Med,* 2002, vol. 8, 793-800
- **IWAI et al.** *PNAS,* 2002, vol. 99, 12293-7
- **GHEBEH et al.** *Neoplasia,* March 2006, vol. 8 (3), 190-8
- **GHEBEH et al.** *BMC Cancer,* 23 February 2008, vol. 8, 57
- **AFANASIEV et al.** *Clin Cancer Res.,* 01 October 2013, vol. 19 (19), 5351-60
- **IWAI et al.** *Int Immunol.,* February 2005, vol. 17 (2), 133-44
- **LYFORD-PIKE et al.** *Cancer Res.,* 15 March 2013, vol. 73 (6
- **CAPELOZZI, V.** *J Bras Pneumol.,* 2009, vol. 35 (4), 375-382
- **WURCH et al.** Development of Novel Protein Scaffolds as Alternatives to Whole Antibodies for Imaging and Therapy: Status on Discovery Research and Clinical Validation. *Current Pharmaceutical Biotechnology,* 2008, vol. 9, 502-509
- **BOBROW, M. N. ; HARRIS, T. D. ; SHAUGHNESSY, K. J. ; LITT, G. J.** *J. Immunol. Methods,* 1989, vol. 125, 279-285